(19) Europäisches Patentamt
European Patent Office
Office européen des brevets

(11) EP 3 285 781 B1

(12) **FASCICULE DE BREVET EUROPEEN**

(45) Date de publication et mention de la délivrance du brevet:
**13.11.2019 Bulletin 2019/46**

(21) Numéro de dépôt: **16721203.4**

(22) Date de dépôt: **22.04.2016**

(51) Int Cl.:
***A61K 31/722*** *(2006.01)* ***A61P 19/00*** *(2006.01)*

(86) Numéro de dépôt international:
**PCT/FR2016/050953**

(87) Numéro de publication internationale:
**WO 2016/170284 (27.10.2016 Gazette 2016/43)**

(54) **SOLUTION AQUEUSE HOMOGENE DE CHITOSANE INJECTABLE PRESENTANT UN PH PROCHE DU PH PHYSIOLOGIQUE**

HOMOGENE WÄSSERIGE LÖSUNG VON INJIZIERBAREM CHITOSAN MIT EINEM PH-WERT NAHE DEM PHYSIOLOGISCHEN PH-WERT

HOMOGENEOUS AQUEOUS SOLUTION OF INJECTABLE CHITOSAN HAVING A PH CLOSE TO PHYSIOLOGICAL PH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.04.2015 FR 1553644**

(43) Date de publication de la demande:
**28.02.2018 Bulletin 2018/09**

(73) Titulaire: **BIOXIS Pharmaceuticals**
**69007 Lyon (FR)**

(72) Inventeurs:
- **GUERRY, Alexandre**
  **38490 Les Abrets (FR)**
- **BERTAINA, Frédéric**
  **30320 Poulx (FR)**

(74) Mandataire: **Plasseraud IP**
**66, rue de la Chaussée d'Antin**
**75440 Paris Cedex 09 (FR)**

(56) Documents cités:
**WO-A1-97/09879** **WO-A1-03/042250**
**WO-A1-2013/079646**

- **FAN M ET AL: "Preparation and structure of chitosan soluble in wide pH range", CARBOHYDRATE POLYMERS, APPLIED SCIENCE PUBLISHERS, LTD. BARKING, GB, vol. 78, no. 1, 4 août 2009 (2009-08-04), pages 66-71, XP026283639, ISSN: 0144-8617, DOI: 10.1016/J.CARBPOL.2009.03.031 [extrait le 2009-03-31]**
- **JAE HYUNG PARK ET AL: "Synthesis and Characterization of Sugar-Bearing Chitosan Derivatives: Aqueous Solubility and Biodegradability", BIOMACROMOLECULES, vol. 4, no. 4, 1 juillet 2003 (2003-07-01) , pages 1087-1091, XP055168092, ISSN: 1525-7797, DOI: 10.1021/bm034094r**
- **DATABASE WPI Week 199016 Thomson Scientific, London, GB; AN 1990-119697 XP002751331, & JP H02 69502 A (CI KASEI KK) 8 mars 1990 (1990-03-08)**
- **VARUM K M ET AL: "Water-solubility of partially N-acetylated chitosans as a function of pH: effect of chemical composition and depolymerisation", CARBOHYDRATE POLYMERS, APPLIED SCIENCE PUBLISHERS, LTD. BARKING, GB, vol. 25, no. 2, 1 janvier 1994 (1994-01-01), pages 65-70, XP024147856, ISSN: 0144-8617, DOI: 10.1016/0144-8617(94)90140-6 [extrait le 1994-01-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne le domaine des produits de comblement ou des bio matériaux, injectables chez l'homme ou l'animal. En particulier, la présente invention concerne une solution aqueuse homogène de chitosane injectable présentant un pH le plus proche possible du pH physiologique (pH=7). La présente invention concerne également des compositions contenant une telle solution aqueuse homogène de chitosane. L'invention a également pour objet de telles compositions pour leur utilisation comme composition dermatologique, cosmétique, ou encore comme dispositif médical, avantageusement comme implant biorésorbable.

Différents produits de comblement injectables notamment chez l'être humain sont déjà connus.

Le collagène a longtemps été le produit de choix comme produit de comblement pour le visage, en particulier pour le comblement des rides et des ridules ou encore pour réourler les lèvres. Cependant, depuis la mise sur le marché des acides hyaluroniques, ces derniers sont les plus utilisés. En effet, à la biodégradabilité du collagène jugée trop rapide, s'ajoutent les problèmes de sécurité liés à l'origine animale (bovine ou porcine) de celui-ci.

L'injection directe d'acide hyaluronique présente deux avantages : un effet de comblement mécanique immédiat et une absence de phénomènes inflammatoires, du fait de sa biocompatibilité. Toutefois, cette biocompatibilité va de pair avec une biodégradation rapide qui rend le produit insatisfaisant, même si la durée de vie du produit injecté a pu être prolongée grâce à l'utilisation d'acide hyaluronique réticulé.

Néanmoins, les produits les plus utilisés aujourd'hui en médecine et chirurgie esthétique sont des produits résorbables dont la durée de vie est généralement inférieure à 12 mois.

On trouve encore sur le marché des produits de comblement qu'on peut qualifier de « permanents », dans le sens où leur biorésorption peut nécessiter plusieurs années. Ces produits contiennent entre autres des polymères synthétiques ou biosynthétiques, tels que des dérivés acryliques, des polyacrylamides, qui induisent une encapsulation fibreuse importante à l'origine de la longévité du comblement. Toutefois, la persistance du produit injecté dans les tissus présente un risque de complications à long terme ou de phénomènes inflammatoires à retardement, par exemple la formation de granulomes inflammatoires, kystes... plusieurs mois, voire plusieurs années, après leur injection. D'autres produits constituent aujourd'hui une alternative intéressante : il s'agit de PLA (acide polylactique), un polymère dont la biodégradation est plus lente que celle des autres polymères naturels, tels que le collagène ou l'acide hyaluronique. On estime en effet que le comblement persiste jusqu'à deux ans après l'injection. Ces produits sont commercialisés notamment sous le nom de New-Fill (ou Sculptra). Le principal défaut de cette technologie est que l'effet de comblement n'est visible qu'après un délai de huit semaines, ce qui n'apporte pas une complète satisfaction au patient.

Par ailleurs, la fibrose observée lors de l'utilisation de produits non dégradables est apparue de grand intérêt en terme d'effet esthétique à long terme, et c'est ainsi qu'ont été développés des produits de comblement qualifiés de « semi-permanents » qui, par leur composition hétérogène « particules-vecteur» ont un effet pro-fibrotique tout en demeurant biodégradables. On citera par exemple le produit Atlean qui propose une dispersion de particules de TCP (phosphate tricalcique) dans de l'acide hyaluronique et le produit Radiesse qui propose une dispersion de particules d'hydroxyapatite de calcium dans un gel de carboxyméthyle cellulose. Dans tous les cas, le gel vecteur assure l'effet esthétique de comblement immédiat, tandis que les particules génèrent petit à petit une fibrose qui garantit l'effet à long terme. L'intérêt de ces produits, outre ce double mécanisme d'action (mécanique et inducteur de tissu), est qu'ils sont finalement totalement résorbés.

De manière particulièrement avantageuse, le chitosane, de par sa structure chimique unique, se comporte vis-à-vis de l'organisme comme un « leurre » du milieu biologique (A. Montembault, K. Tahiri, C. Korwin-Zmijowska, X, Chevalier, M. Corvol, A.Domard, Biochimie, 88 (2006), 551-64): d'une part, il est suffisamment « reconnu » pour ne pas induire de réaction inflammatoire dangereuse, et d'autre part suffisamment « méconnu » pour ne pas être dégradé trop rapidement. La molécule est en effet constituée d'une succession de fragments N-acétyl-D-Glucosamine et D-glucosamine, le premier étant un constituant de molécules de la matrice extracellulaire (on trouve ce résidu dans l'acide hyaluronique par exemple), et le second étant complètement absent de celle-ci, le chitosane est donc plus difficile à dégrader d'un point de vue biologique.

Le concept de l'utilisation d'un « leurre » du milieu biologique est tout à fait nouveau dans le domaine des injectables notamment en médecine esthétique, et aucun produit de comblement contenant du chitosane n'est commercialisé à ce jour.

Par ailleurs, le chitosane est connu dans la littérature pour stimuler certaines cellules de l'immunité, telles que les macrophages, qui produisent en sa présence une quantité accrue de facteurs de croissance. Ces facteurs de croissance sont des médiateurs biologiques qui favorisent la production de matrice extracellulaire et la prolifération des fibroblastes, cellules productrices des fibres de collagène. Ainsi, le chitosane favorise la synthèse de tissu fibreux, ce qui permet un comblement « biologique » à long terme et sans effets secondaires indésirables, la constitution ou la substitution de tissus biologiques ou le comblement des tissus biologiques, par exemple le comblement de dépression cutanée, l'injection dans les cartilages osseux ou dans les articulations).

La demande internationale WO 2013/079646, déposée par la demanderesse, propose notamment une solution

aqueuse homogène de chitosane injectable contenant un chitosane ayant un degré d'acétylation inférieur à 20%, ladite solution aqueuse présentant un pH inférieur à 6,2. Ces formulations présentent de très bons résultats et de bonnes propriétés d'injectabilité à ces pH inférieurs à 6,2. Toutefois, comme expliqué dans cette demande, il est nécessaire de maintenir un pH inférieur à 6,2 pour éviter la gélification de ces solutions. En effet, un chitosane présentant un degré d'acétylation inférieur à 20% tel que celui utilisé dans ces formulations injectables est sensible au pH, et présente la particularité d'être soluble sous forme liquide en solution aqueuse à des pH inférieurs à 6,2, mais de gélifier au-delà d'un pH de 6,2.

Toutefois, lorsqu'on souhaite injecter une solution de chitosane directement dans les tissus qui se trouvent au pH physiologique (entre 6,8 et 7,4), il serait souhaitable d'utiliser une solution présentant un pH le plus proche possible du pH physiologique afin d'éviter la nécrose des tissus du fait de l'acidité de la formulation injectée.

Le document WO 2009/150651 propose quant à lui de formuler des compositions à base de chitosane dont le pH est proche du pH physiologique en associant, dans un hydrogel injectable, un chitosane hautement acétylé soluble au pH physiologique, à un chitosane hautement déacétylé qui précipite à un pH d'environ 6,5 de manière à obtenir un mélange de chitosanes de degrés d'acétylation différents, ledit mélange demeurant ne précipitant pas à un pH supérieur à 6,5. Toutefois, ces hydrogels de texture gélifiée présentent, du fait de leur viscosité plus élevée, de mauvaise propriétés d'injectabilité (ou seringuabilité, c'est-à-dire facilité d'injection du fait d'un écoulement plus ou moins satisfaisant à travers une aiguille dans une seringue) par rapport à des compositions sous forme liquides non gélifiées. En outre, le mélange de deux chitosanes de degrés d'acétylation différents n'est pas simple à mettre en œuvre et peut poser des problèmes d'homogénéité des hydrogels obtenus.

La demande WO03/042250 propose quant à elle de modifier chimiquement par réticulation des chitosanes en solution présentant un pH de l'ordre de 6,8, lesdites solutions étant préparées et maintenues sous forme liquide à des températures très basses (4°C), pour permettre leur gélification à 37°C. C'est donc d'une part la modification chimique du chitosane, et d'autre part, une augmentation de la température, qui permet de contrôler la gélification des solutions à 37°C. Toutefois, dans le cadre de cette demande, des solutions neutres de chitosane ne peuvent être obtenues qu'en maintenant les solutions à une température très basse (de l'ordre de 4 à 5°C), et en utilisant un tampon de type glycérophosphate, connu pour augmenter la solubilité des chitosanes dans l'eau et conférer à la solution de chitosane des propriétés thermogélifiantes. Le glycérophosphate peut toutefois poser des problèmes d'intolérance, notamment lorsqu'il est injecté dans les tissus biologiques. En outre, les solutions liquides neutres obtenues selon la demande WO03/042250 ne peuvent ni être préparées, ni être stockées à température ambiante, ce qui entraine des contraintes et des surcoûts industriels importants.

Il serait donc souhaitable de disposer de formulations à base de chitosane présentant un pH plus proche du pH physiologique, et qui demeurent liquides à température ambiante, en particulier entre 20 et 25°C, pour pouvoir être facilement injectables.

La présente invention a donc pour objet, selon un premier aspect, une solution aqueuse homogène de chitosane injectable contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, ladite solution présentant un pH supérieur ou égal à 6,2, avantageusement compris entre 6,2 et 7,2, ladite solution ne contenant pas de chitosane ayant un degré d'acétylation supérieur à 20%, ladite solution étant liquide et homogène à la température ambiante.

L'invention a également pour objet, selon un second aspect une solution aqueuse telle que décrite précédemment, caractérisée en ce qu'elle est susceptible d'être préparée par un procédé comprenant au moins les étapes suivantes :

- la dissolution du chitosane dans l'eau par ajout d'acide tel qu'un acide faible, pour obtenir une solution aqueuse homogène de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, , ladite solution présentant un pH inférieur à 6,2,
  ledit acide faible étant avantageusement choisi dans le groupe constitué par l'acide acétique, l'acide glycolique, l'acide lactique, l'acide glutamique, et leurs mélanges, et
- le réajustement du pH par dialyse, de préférence à température ambiante, pour obtenir une solution aqueuse présentant un pH supérieur ou égal à 6,2, avantageusement compris entre 6,2 et 7,2, de préférence entre 6,25 et 7,1.

L'invention a donc également pour objet une méthode de préparation d'une solution aqueuse telle que décrite précédemment, comprenant au moins les étapes suivantes :

- la dissolution du chitosane dans l'eau par ajout d'acide tel qu'un acide faible, pour obtenir une solution aqueuse homogène de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise

entre 100 000 et 1 500 000 g/mol, ladite solution présentant un pH inférieur à 6,2,
ledit acide faible étant avantageusement choisi dans le groupe constitué par l'acide acétique, l'acide glycolique, l'acide lactique, l'acide glutamique, et leurs mélanges, et

- le réajustement du pH par dialyse, de préférence à température ambiante, pour obtenir une solution aqueuse présentant un pH supérieur ou égal à 6,2, avantageusement compris entre 6,2 et 7,2, de préférence entre 6,25 et 7,1.

Les solutions aqueuses homogènes de chitosane selon la présente invention sont stables à la température, notamment jusqu'à 40°C, en particulier entre 20 et 30°C, et ne sont donc pas thermo-gélifiables mais gélifient uniquement lors de l'augmentation de pH. Elles se distinguent donc encore, par cet aspect, des formulations décrites dans la demande de brevet WO03/042250. L'obtention de solutions stables à la température est particulièrement avantageuse pour la mise en œuvre industrielle des solutions de l'invention, et leur stockage.

## Chitosane

Les solutions aqueuses selon l'invention comprennent au moins un chitosane.
Le chitosane est un amino-polysaccaharide généralement obtenu par N-désacétylation de la chitine, polysaccharide très répandu dans la biomasse. La chitine est notamment présente dans les cuticules des arthropodes, l'endosquelette des céphalopodes, les parois cellulaires ou encore la matrice extracellulaire de champignons, levures ou algues.
Avantageusement selon la présente invention, le chitosane est un produit naturel qui provient d'une source animale, par exemple de crustacés du type crabes, crevettes ou calmars, ou d'une source végétale, telle que des champignons ou des algues. Le chitosane et la chitine sont des copolymères linéaires respectivement de 2- acetamido-2-desoxy-D-glucopyranose et de 2-amino-2-desoxy-D-glucopyranose. On parle plus communément d'unités N-acétyl-D-glucosamine (GlcNAc) et D-Glucosamine (GlcN)), liées par des liaisons glycosidiques β-(1→4). Chitine et chitosane se différencient par la fraction molaire (exprimée en %) des unités GlcNAc présentes dans le copolymère, appelée aussi degré d'acétylation (DA).
Les structures chimiques du chitosane et de la chitine sont représentées schématiquement ci-dessous en fonction du degré d'acétylation (DA) :

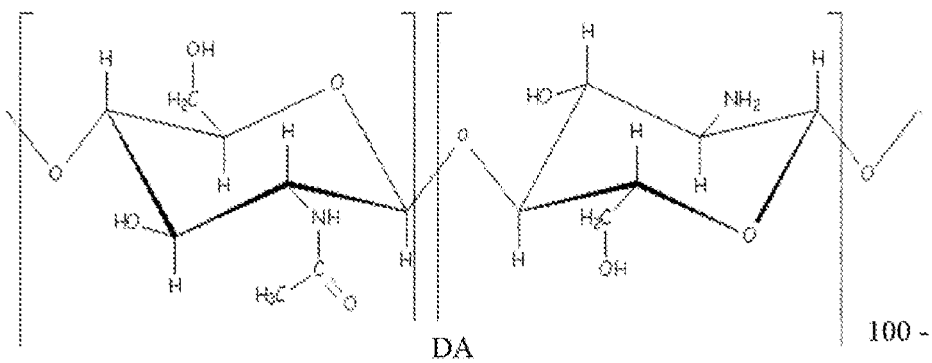

DA 100 - DA

N-acétyl-D-Glucosamine (GlcNAc) D-Glucosamine(GlcN)

Degré d'acétylation (DA) :

$$DA(\%) = \frac{nGlcNAc}{nGlcNAc + nGlcN} \times 100$$

avec nGlcNAc = nombre de motifs acétylés et nGlcN = nombre de motifs désacétylés.
Selon la présente invention, le chitosane a un degré d'acétylation (DA) inférieur à 20%, de préférence inférieur à 19%, plus préférentiellement inférieur à 17%, encore plus avantageusement inférieur ou égal à 15%, par exemple inférieur à 10%.
Typiquement, le chitosane selon l'invention a un degré d'acétylation (DA) compris entre 0,5 et 20 %, de préférence encore entre 1 et 19 %, de préférence encore entre 1 et 17 %, plus préférentiellement entre 2 et 15% et encore plus préférentiellement entre 2 et 10%.

Le degré d'acétylation est de préférence mesuré selon la méthode décrite dans la publication «Physico-chemical studies of the gelation of chitosan in a hydroalcoholic médium » A. MONTEMBAULT, C. VITON, A. DOMARD Biomaterials, 26(8), 933-943, 2005).

Le chitosane mis en œuvre dans les solutions de l'invention a une masse moléculaire moyenne en masse (déterminée avant stérilisation selon la méthode décrite dans «Physico-chemical studies of the gelation of chitosan in a hydroalcoholic médium » A. MONTEMBAULT, C. VITON, A. DOMARD Biomaterials, 26(8), 933-943, 2005) comprise entre 100 000 et 1 500 000 g/mol, avantageusement entre 200 000 et 1 000 000 g/mol, plus avantageusement entre 250 000 et 800 000 g/mol, et encore plus avantageusement entre 300 000 et 600 000 g/mol.

Classiquement un chitosane de masse molaire comprise entre 100 000 à 1 000 000 $g.mol^{-1}$ peut aussi être caractérisé par sa viscosité (Classiquement à une concentration de 1% dans une solution aqueuse à 1% en acide acétique à 25°C). Avec cette considération, la masse molaire du chitosane peut aussi être définie par une viscosité comprise entre 5 Pa.s et 20 Pa.s.

La viscosité de la composition est mesurée à 25 °C à l'aide d'un rhéomètre DHR1 (TA industrie) et une géométrie plan de 40mm selon un mode dynamique avec taux de cisaillement appliqué de 0,01 à 1 $s^{-1}$.

Selon un mode préféré de réalisation, le chitosane mis en œuvre dans les solutions selon l'invention n'est pas modifié chimiquement, et en particulier, n'est pas greffé pour favoriser sa solubilité en solution aqueuse à des pH proches de la neutralité (entre 6,2 et 7,2). Il se distingue en ce sens des chitosanes mis en œuvre dans la demande WO03/042250 ou la demande JP-H02-69502, dans la publication « Synthesis and characterization of sugar-bearing chitosan derivatives : aqueous solubility and biodegradability », Jae Hyung Park et al. , Biomacromolecules 2003, 4, 1087-1091, et dans la publication « Water solubility of partially N-acetylated chitosans as a function of pH : effect of chemical composition and depolymerization » Varum K. M. et al., Carbohydrate polymers 25 (1994), 65-70.

Selon une caractéristique particulière, un autre chitosane de plus faible masse moléculaire moyenne, avantageusement inférieure à 20 000 g/mol, peut-être ajouté au chitosane tel que défini précédemment, sous réserve de ne pas altérer l'homogénéité de la solution ainsi obtenue.

Dans ce mode de réalisation, cet autre chitosane peut se présenter sous forme de particules de chitosane réticulé.

## Solution

La solution aqueuse homogène de chitosane injectable selon l'invention contient, dans un milieu physiologiquement acceptable, un chitosane ayant un degré d'acétylation inférieur à 20% et une masse moléculaire moyenne en masse comprise entre 100 000 et 1 500 000 g/mol, tel que décrit précédemment.

Par « milieu physiologiquement acceptable », on entend, au sens de la présente demande, un milieu qui ne présente aucun risque d'intolérance ou de toxicité lors de l'injection dans les tissus biologiques de la solution injectable selon l'invention. Le milieu physiologiquement acceptable doit donc en particulier être inerte et biocompatible vis-à-vis des tissus biologiques tels que les muscles, les articulations, les globes oculaires, et de façon plus générale dans les tissus mous ou durs du corps par exemple les organes (appareil digestif ou uro-génital) ou les tissus adipeux, les muqueuses, les gencives, le cartilage, les os...

Le pH de la solution aqueuse selon la présente invention est supérieur ou égal à 6,2, et est avantageusement compris entre 6,2 et 7,2, de préférence entre 6,25 et 7,1, et plus préférentiellement entre 6,3 et 7,0.

Dans le cadre de l'invention, le chitosane est soluble en solution aqueuse, telle que l'eau, dans les gammes de pH mentionnées précédemment, avantageusement par protonation des groupes amine du chitosane. Avantageusement, la solution aqueuse selon l'invention est stable, en particulier stable (conservation de ses caractéristiques rhéologiques, de sa couleur, de sa transparence et/ou de sa limpidité à 25°C) au stockage à des températures allant de 4 à 25°C pendant au moins 1 mois, de préférence au moins 6 mois, et plus préférentiellement au moins 24 mois. En d'autres termes, même après un stockage sur une période prolongée à des températures entre 4 et 25°C, ses caractéristiques à 25°C (rhéologiques, sa couleur, sa transparence et/ou sa limpidité) de la solution aqueuse selon l'invention sont préservées.

Par « solution » injectable, on entend au sens de la présente invention une composition se présentant sous forme liquide, par opposition à une composition gélifiée. Elle se distingue ainsi des compositions sous forme d'hydrogels injectables à base de chitosane solubilisé telles que celles décrites dans la demande de brevet WO 2009/150651. Ces hydrogels de texture gélifiée présentent, du fait de leur viscosité plus élevée, de moins bonnes propriétés d'injectabilité (ou seringuabilité, c'est-à-dire facilité d'injection du fait d'un écoulement plus ou moins satisfaisant à travers une aiguille dans une seringue) que les solutions liquides selon l'invention.

On entend par solution « liquide » au sens de la présente invention, une composition qui s'écoule sous son propre poids, en particulier après au plus 24h, de préférence au plus 10h, contrairement à un gel, et notamment un hydrogel.

Les solutions liquides selon l'invention présentent de préférence une viscosité à 25°C inférieure à 1000 Pa.s,

de préférence comprise entre 20 et 800 Pa.s, plus préférentiellement entre 50 et 600 Pa.s. A titre comparatif, les formulations sous forme d'hydrogels présentent généralement une viscosité de l'ordre de 4000 à 10000 Pa.s.

La viscosité de la composition est mesurée à 25 °C à l'aide d'un rhéomètre DHR1 (TA industrie) et une géométrie plan de 40mm selon un mode dynamique avec taux de cisaillement appliqué de 0,01 à 1 $s^{-1}$.

La distinction entre les solutions selon l'invention et des formulations sous forme de gels peut notamment être mise en évidence par la mesure des propriétés rhéologiques de ces compositions.

La distinction gel/solution est réalisée lors d'une étude rhéologique en oscillation de 0,1 à 100 rad.$s^{-1}$ à fréquence constante de 1Hz à 25°C afin de déterminer le modules visqueux G" et le module élastique G'.

En effet, une solution liquide au sens de la présente invention se caractérise notamment par le fait que son module visqueux G" est supérieur à son module élastique G'. Dans le cas d'un gel, au contraire, le module élastique G' est supérieur au module visqueux G". Les mesures sont effectuées sur un rhéomètre DHR2 (TA industries) et une géométrie plan 40 mm selon un mode dynamique (fréquence angulaire : 100 à 1 rad/s, déformation 1 %, 37°C). Les échantillons constitués de 2,5 ml de solution de chitosane à tester sont déposés dans des coupelles, placées à 37°C dans du milieu de culture pendant 24h.

Par solution « homogène » de chitosane, on entend au sens de la présente invention que tout le polymère chitosane est dissous, la solution ne contenant pas de particules solides en suspension dans la phase liquide. La solution selon l'invention est également typiquement transparente. L'homogénéité de la solution de chitosane peut notamment être caractérisée par mesure de la transmittance de la lumière au travers d'un échantillon de solution. Ainsi, selon un mode préféré de réalisation, la solution de chitosane selon la présente invention a une valeur de transmittance de la lumière, à une longueur d'onde égale à 500 nm, à travers un échantillon de 1 cm d'épaisseur, supérieure à 60 %, de préférence supérieure à 70 %, plus préférentiellement supérieure) 80%, et encore plus préférentiellement supérieure) 90%. En particulier, la solution aqueuse homogène selon l'invention présente une transmittance comprise entre 60 et 100%, de préférence entre 70 et 99%, plus préférentiellement entre 80 et 98%, et encore plus préférentiellement entre 90 et 97%.

Les solutions liquides homogènes de chitosane objets de la présente invention sont de préférence liquides et homogènes à la température ambiante, c'est-à-dire entre 20 et 25°C, de préférence à 25°C. Elles se distinguent sur ce point des solutions intermédiaires décrites dans les exemples la demande WO03/042250, qui sont liquides à basse température (4°C) mais gélifient et se troublent lors de l'augmentation de la température.

Les solutions liquides homogènes de chitosane objet de la présente invention sont de préférence stables au stockage à 25°C dans le temps. En particulier, leur viscosité à 25°C et leur transmittance restent stables pendant au moins 1h et jusqu'à plus de 3 ans pour permettre le stockage des solutions. Ainsi, les solutions selon l'invention présentent de préférence une stabilité au stockage à 25°C pendant une durée comprise entre 1h et 3 ans, plu préférentiellement entre 10h et 2 ans, encore plus préférentiellement entre 1 jour et 1 an, et plus préférentiellement encore entre 1 mois et 9 mois.

Selon une caractéristique particulière de la présente invention, la solution aqueuse homogène de chitosane contient entre 0,1 et 4,5 %, avantageusement entre 1 et 3,5 %, en particulier entre 2 et 3,5 %, en poids de chitosane, par rapport au poids total de la solution aqueuse.

De manière particulièrement avantageuse, la solution aqueuse selon l'invention est formulée pour être administrée ou est utilisée par injection par voie intradermique ou sous-cutanée, intramusculaire, intra articulaire, intra oculaire, et de façon plus générale dans les tissus mous ou durs du corps par exemple les organes (appareil digestif ou urogénital) ou les tissus adipeux, les muqueuses, les gencives, le cartilage, les os, etc. Du fait de son pH proche du pH physiologique, la solution selon l'invention peut être injectée directement dans les tissus sans risquer d'entrainer leur nécrose. La solution peut être conditionnée dans une seringue telle qu'une seringue stérile.

Dans un mode de réalisation particulier, la solution aqueuse selon l'invention est stérilisée avant injection, par exemple par autoclave.

Après stérilisation, le chitosane a typiquement une masse moléculaire moyenne en masse comprise entre 50 000 et 1 200 000 g/mol, avantageusement comprise entre 100 000 et 1 000 000 g/mol.

Après stérilisation, les solutions liquides selon l'invention présentent de préférence une viscosité inférieure à 800 Pa.s, de préférence comprise entre 5 et 800 Pa.s. La viscosité des solutions après stérilisation est mesurée selon la méthode décrite pour mesurer la viscosité des solutions avant stérilisation.

De manière particulièrement avantageuse, la solution aqueuse selon la présente invention avant injection ne contient pas de chitosane ayant un degré d'acétylation supérieur à 20%. Ainsi, le chitosane selon l'invention n'est pas mélangé avec un chitosane ayant un degré d'acétylation compris entre 30 et 60%, tel que cela est décrit dans les demandes de brevet WO 2008/072230 et WO 2009/150651.

Dans un mode de réalisation particulier selon la présente invention, la solution aqueuse contient plusieurs chitosanes, mais avec un seul degré d'acétylation (DA), ledit degré d'acétylation étant inférieur à 20%, avantageusement inférieur à 10%.

Dans un autre mode de réalisation particulier de la présente invention, la solution aqueuse contient un chitosane

tel que défini précédemment en mélange avec un autre chitosane, tel qu'un oligosaccharide de chitosane encore appelé chito-oligosaccharide, ayant un degré d'acétylation inférieur à 20%, avantageusement inférieur à 10%, de manière encore plus avantageuse ayant un degré d'acétylation identique au chitosane tel que défini précédemment, et ayant typiquement une très faible masse moléculaire moyenne en masse, par exemple inférieure à 20 000 g/mol, avantageusement inférieure à 17 000 g/mol.

Dans un autre mode de réalisation particulier de la présente invention, la solution aqueuse contient, à titre de polymère, un seul chitosane ayant un degré d'acétylation tel que défini précédemment, ayant avantageusement une masse moléculaire moyenne telle que définie précédemment, avantageusement en une teneur comprise entre 0,1 et 4,5 %, avantageusement entre 1 et 3,5 %, en particulier entre 2 et 3,5 %, en poids de chitosane, par rapport au poids total de la solution aqueuse.

## Agent réticulation

Dans un mode de réalisation particulier, le chitosane peut être partiellement réticulé par interactions ioniques induites par exemple par l'ajout de sulfate, citrate, anions métalliques ou encore molécules anioniques, en particulier par la formation de complexes polyélectrolytes avec des polysaccharides présentant un groupe carboxylique $COO^-$ (alginates, pectine, xanthane, acide hyaluronique), avec des polysaccharides possédant un groupe sulfate, ou avec l'acide polylactique (PLA), ou encore par interaction avec des protéines (le collagène), des acides nucléiques (l'ADN, l'ARN, les Si ARN, les mARN...) ou des polysaccharides oxydés.

Dans un autre mode de réalisation particulier, le chitosane peut être partiellement réticulé à l'aide d'agents réticulants covalents (ex : génipine), à l'exclusion d'agents connus pour leur toxicité, tels que des agents du groupe des époxy (par exemple le 1,4-butanediol diglycidyl ether - BDDE) ou esters bi ou poly fonctionnels (par exemple l'Éthylène Diamine Tétra-Acétique - EDTA), de la divinyl sulfone, des carbodiimides, et des dialdéhydes.

Dans un mode de réalisation particulier, la solution aqueuse selon l'invention est composée de l'association d'une solution aqueuse de chitosane non réticulée avec une solution aqueuse de chitosane réticulée.

## Procédé de préparation de la solution

Selon une caractéristique particulière, la solution aqueuse selon l'invention est susceptible d'être préparée par les étapes suivantes

- la dissolution du chitosane dans l'eau par ajout d'acide tel qu'un acide faible pour obtenir une solution aqueuse homogène de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol ladite solution présentant un pH inférieur à 6,2,
  ledit acide étant avantageusement choisi dans le groupe constitué par l'acide acétique, l'acide glycolique, l'acide lactique, l'acide glutamique, et leurs mélanges, et
- le réajustement du pH par dialyse, de préférence à température ambiante, pour obtenir une solution aqueuse présentant un pH supérieur ou égal à 6,2, avantageusement compris entre 6,2 et 7,2, de préférence entre 6,25 et 7,1.

Avant dissolution, le chitosane est typiquement sous forme de poudre ou de paillette. Après dissolution, le chitosane est sous forme protonée. Il s'agit d'un polyélectrolyte cationique dont le contre-ion est issu de l'acide utilisé pour la dissolution. Par exemple, si l'acide acétique est ajouté à l'eau pour dissoudre le chitosane, on retrouve le chitosane sous forme d'acétate de chitosane, c'est-à-dire une forme protonée $NH_3^+$ de fonctions aminées en interaction électrostatique avec les ions acétates.

Le chitosane est dissous dans l'eau à l'aide d'un acide fort ou d'un acide faible. Toutefois, le chitosane est de préférence dissous dans l'eau à l'aide d'un acide faible pour éviter sa dégradation par hydrolyse en solution acide (à des pH de l'ordre de 3 ou moins obtenus lors de la solubilisation par acide fort). Les acides forts, bien que permettant effectivement la solubilisation du chitosane dans l'eau, sont plus difficiles à mettre en œuvre et imposent de travailler précisément à la stœchiométrie.

L'acide fort peut par exemple être l'acide chlorhydrique ou l'acide phosphorique.

L'acide faible peut être choisi parmi l'acide acétique, l'acide glycolique, l'acide lactique, l'acide glutamique, et leurs mélanges.

Dans un mode de réalisation particulier selon l'invention, lors de l'étape de dissolution, l'acide est ajouté en une quantité strictement nécessaire à la dissolution du chitosane. On peut utiliser ainsi un excès d'acide pour certains chitosanes, par exemple les chitosane difficiles à solubiliser avec la quantité strictement nécessaire d'acide, puis on reprécipite le chitosane, à l'aide d'ammoniaque par exemple. Après une série de lavages destinés à éliminer l'excédent d'ammoniaque et les sels, on peut alors lyophiliser le chitosane pour récupérer la matière sèche. Celle-ci sera alors plus

facile à solubiliser.

Dans un autre mode de réalisation particulier selon l'invention, lors de l'étape de dissolution, l'acide est ajouté en une quantité strictement nécessaire à la dissolution du chitosane, telle que la quantité stœchiométrique strictement nécessaire à la protonation des sites $NH_2$.

Typiquement, le nombre de sites à protoner est calculé de la manière suivante :

$$M_{monomère} = 203 \text{ x } DA + 161 \text{ x } (1 - DA)$$

$$n_{NH_2} \frac{m \times (1 - DA) \times (1 - \%_{H_2O})}{M_{monomère}}$$

avec m = masse de matière première introduite, %eau = teneur en eau de la matière première, DA = degré d'acétylation, $n_{NH_2}$ = le nombre de site à protoner en mol. $M_{monomère}$ = Masse molaire moyenne des résidus en $g.mol^{-1}$

Le contrôle du pH des solutions est très important pour éviter la nécrose acide des tissus après injection, et également pour protéger les solutions de l'hydrolyse et la dégradation du chitosane si l'on met en œuvre une stérilisation (par exemple par autoclave à 121°C pendant 15 minutes).

Dans la cadre de la présente invention, l'ajustement du pH se fait de manière très progressive par dialyse.

Sans vouloir être lié par une quelconque théorie, il semble qu'en ajustant le pH de manière progressive (notamment par dialyse), par opposition à un ajustement brutal par adjonction d'une solution tampon, il est possible de maintenir la solution sous forme liquide non gélifiée tout en atteignant des valeurs de pH plus proche du pH physiologique. Les procédés de l'art antérieur dans lesquels l'ajustement du pH est opéré par ajout d'un composé tel que le bicarbonate de sodium ou d'un tampon PBS (« Phosphate Buffer Saline » - solution saline de tampon phosphate), semblent entrainer une augmentation trop brusque du pH, conduisant à une gélification de la solution dès un pH de 6,2. Contre toute attente, les inventeurs à l'origine de la présente demande ont mis en évidence qu'en contrôlant la remontée de pH pour la rendre continue, et progressive, il était possible de maintenir la solution aqueuse de chitosane sous forme liquide à des pH supérieurs à 6,2, s'approchant encore davantage du pH physiologique.

La valeur du pH est avantageusement contrôlée grâce à un pH-mètre pendant la remontée de pH pour atteindre à un pH supérieur ou égal à 6,2 et avantageusement compris entre 6,2 et 7,2, de préférence entre 6,25 et 7,1, et plus préférentiellement 6,3 et 7,0.

La dialyse est un procédé de séparation par membrane des molécules ou des ions en solution. Ainsi, dans le cadre de la présente demande, la solution aqueuse de chitosane selon l'invention peut être dialysée contre une solution tampon présentant un pH égal au pH final souhaité pour la solution de chitosane (pH cible), c'est à-dire au moins supérieur à 6,2, avantageusement compris entre 6,2 et 7,2, de préférence entre 6,25 et 7,1. Lorsque la solution tampon présente un pH supérieur au pH de gélification de la solution (par exemple 7,5), la dialyse devrait alors être surveillée pour éviter la gélification de la solution.

La solution tampon peut, par exemple, être une solution saline de tampon phosphate (PBS, TBS, PBS-acide lactique), la tris (tris(hydroxymethyl)methylamine), 4-2-hydroxyethyl-1-piperazineethanesulfonic acid (HEPES), 2-{[tris(hydroxymethyl)methyl]amino}ethanesulfonic acid (TES), 3-(N-morpholino)propanesulfonic acid (MOPS), piperazine-N,N'-bis(2-ethanesulfonic acid)), MES (2-(N-morpholino)ethanesulfonic acid (PIPES), du chlorure de sodium (NaCl).

Selon un mode préféré de réalisation, la solution tampon est une solution de tampon phosphate PBS (« Phosphate Buffer Saline » - solution saline de tampon phosphate) composée d'un sel « acide » $NaH_2PO_4$, d'un sel «basique » le $Na_2HPO_4$ et de NaCl.

Selon un mode particulier de réalisation, le tampon est physiologiquement acceptable, c'est-à-dire qu'il ne présente aucun risque d'intolérance ou de toxicité lors de l'injection dans les tissus de la solution injectable selon l'invention. A ce titre, le tampon est de préférence différent du glycérophosphate, et en particulier du β-glycérophosphate qui, bien que peu irritant pour la peau, pose des problèmes de calcification lorsqu'il est injecté dans les tissus.

Selon un mode de réalisation particulier de l'invention, la dialyse peut être réalisée de manière statique dans un seul bain contre une solution tampon telle que décrite précédemment.

Dans ce mode de réalisation, la solution tampon peut présenter un pH supérieur au pH souhaité pour la solution de chitosane, par exemple entre 7,0 et 7,5. La dialyse doit alors être surveillée en temps réel pour ne pas rehausser le pH au-delà du pH de gélification. Toutefois, l'utilisation d'une solution tampon présentant un pH trop élevé peut présenter l'inconvénient de créer un gradient de pH au sein de la poche de dialyse comprenant la solution aqueuse de chitosane, notamment entre la périphérie de la poche et son centre, ce qui pourrait altérer l'homogénéité de la solution de chitosane. De plus, si ce gradient de pH entraine une gélification de la solution aqueuse de chitosane en périphérie de la membrane, cela pourrait diminuer l'efficacité et la qualité de la dialyse en limitant les échanges de proton, de sel et de solvant entre la solution de chitosane et la solution tampon.

Ainsi, lorsque la dialyse est réalisée de manière statique dans un seul bain contre une solution tampon, il est préférable que ladite solution tampon présente un pH égal au pH final souhaité pour la solution de chitosane (pH cible), par exemple entre 6,5 et 6,9.

Selon un mode plus préféré de réalisation, la dialyse peut être réalisée de manière statique dans plusieurs bains successifs contre des solutions tampon présentant des pH différents de plus en plus proches du pH final souhaité pour la solution de chitosane (pH cible). Il est ainsi possible de rehausser le pH de manière plus progressive en fonction du nombre de bains tampons mis en œuvre, de manière à tendre le plus possible vers le pH de gélification de la solution de chitosane sans jamais l'atteindre. Cependant, chaque changement de bain s'accompagne d'une variation brusque de pH, quoique de faible amplitude, qui est susceptible de déstabiliser la solution de chitosane et d'entrainer sa gélification à des pH de l'ordre de 6,3-6,4. Ces pH sont certes plus élevés que ceux obtenus pour les compositions de l'art antérieur mettant en œuvre l'adjonction d'un tampon directement dans la solution aqueuse de chitosane, mais pourraient s'approcher davantage du pH physiologique pour minimiser les risques de nécrose des tissus lors de l'injection de la solution aqueuse de chitosane.

Ainsi, selon un mode particulièrement préféré de réalisation, la dialyse est réalisée de manière dynamique, c'est-à-dire en faisant circuler en continu au moins une solution permettant l'augmentation progressive du pH au travers d'une ou plusieurs poches de dialyse constituées d'une membrane de dialyse renfermant la solution aqueuse de chitosane.

Ce procédé de dialyse dynamique met notamment en œuvre un premier réservoir A contenant un volume $V_A$ de solution d'acide faible. La solution acide faible contenue dans le réservoir A est mise à circuler au travers d'une série de poches de dialyse reliées en série ou en parallèle les unes avec les autres. La sortie de la dernière poche est reliée à un second réservoir B contenant un volume $V_B$ de base, ladite base pouvant être la base conjuguée de l'acide faible mis en œuvre dans le premier réservoir A, ou une base plus forte que celle-ci. Le contenu du second réservoir B se déverse ensuite dans le réservoir A, modifiant ainsi sa composition. Le système fonctionne en circuit fermé jusqu'à ce que l'équilibre acido-basique soit atteint dans les réservoirs A et B, formant ainsi, dans chaque réservoir, une solution tampon de pH déterminé. De cette manière, le pH de la solution de chitosane contenue dans les poches de dialyse augmente progressivement jusqu'à atteindre celui de la solution tampon choisie. Un tel dispositif est notamment illustré à la Figure 1.

Ainsi, le procédé de dialyse dynamique selon l'invention met notamment en œuvre les étapes suivantes:

i. la préparation, dans un premier réservoir A, d'une première solution comprenant un acide faible, appelée « solution acide faible »,

ii. la préparation, dans un second réservoir B, d'une seconde solution comprenant la base conjuguée ou une base plus forte que la base conjuguée de l'acide faible utilisé dans la « solution acide faible »,

iii. la circulation de la « solution acide faible » au travers d'une ou plusieurs poches de dialyse constituées d'une membrane de dialyse renfermant une solution aqueuse de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, , ladite solution présentant un pH inférieur à 6,2,

iv. l'introduction de ladite « solution acide faible » récupérée à l'issue de la dialyse de l'étape iii) dans le second réservoir B, la composition dudit second réservoir B étant ainsi modifiée en un mélange « base + $\varepsilon$ acide faible », $\varepsilon$ représentant une quantité très minoritaire,

v. l'introduction du mélange « base + $\varepsilon$ acide faible » du réservoir B dans le réservoir A pour obtenir un mélange « acide faible + $\varepsilon$ base» dans ledit réservoir A,

vi. la répétition des étapes iii, iv, et v jusqu'à ce que l'équilibre acido-basique soit atteint dans les réservoirs A et B, formant ainsi, dans chaque réservoir, une solution tampon de pH déterminé.

La solution acide faible contenue dans le réservoir A peut notamment comprendre tout acide faible, de préférence plus faible que l'acide mis en œuvre pour la dissolution du chitosane.

De préférence, l'acide faible mis en œuvre dans la solution du réservoir A est choisi parmi le dihydrogénophosphate ($H_2PO_4^-$), l'acide 4-(2-hydroxyéthyl)-1-pipérazine éthane sulfonique, l'acide 2-[[1,3-dihydroxy-2-(hydroxymethyl)propan-2-yl]amino]ethanesulfonique, l'cide 3-(N-morpholino)propanesulfonique, l'acide piperazine-N,N'-bis(2-ethanesulfonique) l'acide 2-(N-morpholino)ethanesulfonique, les acides carboxyliques tels que l'acide acétique, l'acide glycolique. De préférence encore, l'acide faible mis en œuvre dans le réservoir A est un mélange de dihydrogénophosphate ($H_2PO_4^-$) et de NaCl.

Pour contrôler simultanément l'osmolarité de la solution de chitosane, l'acide faible mis en œuvre dans la solution du réservoir A peut être associé à un sel dit neutre, c'est-à-dire n'interagissant pas avec le couple acide base contenu dans les réservoirs A et B, notamment un de sel de sodium (NaCl) ou de potassium (KCl).

La solution basique contenue dans le réservoir B comprend la base conjuguée ou une base plus forte que la

base conjuguée de l'acide faible utilisé dans la solution acide faible utilisé dans la solution du réservoir A. Ainsi, lorsque le dihydrogénophosphate ($H_2PO_4^-$) est l'acide faible mis en œuvre dans le réservoir A, l'hydrogénophophate ($HPO_4^{2-}$) peut être utilisé dans le réservoir B. Le couple acide-base est choisi de manière à ce que son pKa soit proche du pH souhaité pour la solution tampon (+/- 1). En particulier, le pKa du couple acide-base peut être avantageusement compris entre 5,3 et 8,2, de préférence entre 5,5 et 7,5.

Les poches de dialyse sont constituées d'une membrane de dialyse renfermant la solution aqueuse de chitosane. La membrane peut par exemple être constituée d'un polymère de cellulose régénérée (cellulose naturelle transformée, par une suite d'opérations chimiques et physiques) ou d'un ester de cellulose (par exemple l'acétate de cellulose) avec un seuil d'exclusion compris entre 5kDa et 30KDa.

Un système de pompes peut notamment permettre d'assurer la circulation en continu de la solution d'acide faible contenue dans le réservoir A et de base conjuguée contenue dans le réservoir B.

Un système d'agitation peut être mis en place dans les deux réservoirs A et B. Dans le réservoir A, il permet d'homogénéiser la solution et d'éviter les variations de pH liées à l'introduction d'une partie ($\varepsilon$) de la solution contenue dans le réservoir B. Dans le réservoir B, l'agitation est particulièrement utile lorsque la base mise en œuvre est sous forme de sel. L'agitation permet ainsi de solubiliser progressivement le sel basique. L'utilisation d'une base sous forme de sel permet donc de ralentir encore la remontée de pH et donc d'assurer une dialyse encore plus progressive.

A l'issue du procédé de dialyse dynamique, l'équilibre acido-basique est atteint dans les réservoirs A et B, formant ainsi, dans chaque réservoir, une solution tampon AB de pH déterminé.

La teneur en acide faible mis en œuvre dans le réservoir A et la teneur en base conjuguée mises en œuvre dans le réservoir B sont calculées par des méthodes connues de l'homme du métier pour obtenir, après rétablissement de l'équilibre acide-basique, une solution tampon AB présentant le pH souhaité.

Le volume totale de la solution tampon AB ($V_{AB}=V_A+V_B$) représente de préférence au moins 5 fois le volume de solution aqueuse de chitosane à dialyser, de préférence au moins 10 fois.

Le volume $V_A$ de solution d'acide faible représente de préférence 100 fois le volume $V_B$ de base conjuguée ou de base plus forte que la base conjuguée de l'acide faible mis en œuvre dans le premier réservoir A. En effet, plus la base mise en œuvre dans le réservoir B est concentrée ou forte, plus la dialyse est rapide. Pour ralentir la dialyse, on préfère, dans le cadre de l'invention, augmenter le volume $V_B$ du réservoir B à volume Va du réservoir A constant.

La circulation en continu des solutions A et B, précurseurs de la solution tampon AB, permettent une augmentation progressive du pH (et de l'osmolarité lorsque des sels sont mis en œuvre) de la solution de chitosane permettant d'approcher les valeurs physiologiques. La cinétique d'ajustement du pH peut être contrôlée en modifiant la vitesse de circulation des solutions A et B.

Le procédé de dialyse dynamique présente l'un au moins des avantages suivants:

- éviter la formation d'un film de chitosane gélifié sur la membrane de dialyse
- ne pas provoquer de rupture physico chimique dans la solution de chitosane qui pourrait compromettre sa stabilité
- tendre plus facilement vers le pH de gélification sans provoquer cette dernière ; et approcher au plus près cette zone
- augmenter la reproductibilité des essais
- s'affranchir du contrôle permanent du pH pendant la dialyse
- augmenter l'homogénéité de la solution de chitosane et donc des seringues après répartition, facilitant la stérilisation (contenu identique dans chaque seringue).
- limiter la manipulation des membranes de dialyse et donc de limiter les risques de contamination
- limiter les interventions humaines et donc le coût de la dialyse.

Après injection notamment dans les tissus, la solution aqueuse homogène selon la présente invention va former avantageusement un système semi- cristallin, en particulier du fait du changement de pH lié à l'influence des milieux tamponnés de l'organisme.

Par « système semi-cristallin », on entend typiquement un système constitué d'une phase cristalline et d'une phase non cristalline (amorphe).

Typiquement, les cristaux de chitosane obtenus correspondent à l'allomorphe hydraté du chitosane.

De manière particulièrement avantageuse selon la présente invention, la solution aqueuse présente une bonne biocompatibilité et est biorésorbable. En particulier, le produit selon l'invention a une durée de biorésorption suffisamment longue, pour un effet prolongé, tel qu'un effet de comblement prolongé.

Par « biorésorbable » ou « biorésorption », on entend une biodégradation qui aboutit à la dégradation totale ou essentiellement totale du produit injecté.

Selon une caractéristique particulière de la présente invention, la solution de chitosane est fluide avant injection, gélifie rapidement (en quelques minutes à quelques heures) *in situ,* et présente un temps de résorption long une fois injectée, typiquement de quelques semaines à plusieurs mois, par exemple de l'ordre de 3 ou 4 semaines jusqu'à 12 à 18 mois. Le produit ou biomatériau constitué de ou contenant la solution aqueuse selon l'invention bénéficie du caractère

bactério et fongistatique du chitosane, bien connu dans le monde de l'industrie agro-alimentaire et des pansements cicatrisants. Ces propriétés facilitent la conservation du produit et contribuent à limiter les risques d'infection liés à l'injection ou les phénomènes inflammatoires à retardement pour d'autres produits tels qu'évoqués ci-dessus. Face aux molécules naturelles utilisées à ce jour pour le comblement de rides (collagène, acide hyaluronique), le chitosane est le seul à présenter de telles propriétés. Par ailleurs, le produit ou biomatériau constitué de ou contenant la solution aqueuse selon l'invention assure un comblement biologique efficace avantageusement immédiat: le chitosane favorisant en effet la synthèse de collagène permet un comblement des défauts cutanés, tels que les rides, par stimulation de mécanismes naturels.

La présente invention a également pour objet une solution aqueuse homogène de chitosane injectable contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, , ladite solution présentant un pH supérieur ou égal à 6,2, avantageusement compris entre 6,2 et 7,2, ladite solution ne contenant pas de chitosane ayant un degré d'acétylation supérieur à 20%, ladite solution étant liquide et homogène à la température ambiante, pour son utilisation dans la constitution ou la substitution de tissus biologiques, par exemple comme implant, ou le comblement des tissus biologiques, par exemple le comblement de dépression cutanée, l'injection dans les cartilages osseux ou dans les articulations).

Avantageusement, la solution et le chitosane sont tels que définis précédemment.

En particulier, le chitosane a une masse moléculaire moyenne en masse comprise entre 100 000 et 1 500 000 g/mol, avantageusement entre 200 000 et 1 000 000 g/mol, plus avantageusement entre 250 000 et 800 000 g/mol, et encore plus avantageusement entre 300 000 et 600 000 g/mol.

Typiquement, ladite solution aqueuse ne contient pas de chitosane ayant un degré d'acétylation supérieur à 20%.

De manière avantageuse, ladite solution aqueuse est susceptible d'être préparée selon les étapes du procédé mentionné précédemment.

La présente invention a également pour objet une composition comprenant une solution aqueuse selon l'invention, et éventuellement un composé ou un excipent acceptable.

Dans un mode de réalisation particulier, la solution aqueuse de chitosane selon l'invention comprend un sel tel que le chlorure de sodium, ou tout autre excipent acceptable avantageusement pour ajuster l'osmolarité de la composition. L'ajout d'un sel tel que le chlorure de sodium peut être intéressant pour obtenir une solution isotonique.

Selon une caractéristique particulière de la présente invention, la composition peut comprendre en outre au moins un composé ayant une activité thérapeutique reconnue. On peut citer à titre d'exemple un composé analgésique, un composé anesthésique local comme la lidocaïne, mépivacaïne, bupivacaïne ou ropivacaïne, un composé angiogénique, un vaccin, une hormone, ou encore un composé actif du type facteur de croissance ou oligosaccharide bioactif, par exemple un oligosaccharide d'acide hyaluronique ou oligosaccharide de chitosane de degré de polymérisation inférieur à 20, ou encore un acide nucléique, une protéine ou un anticancéreux.

La présente invention a également pour objet une telle composition ou une solution aqueuse selon l'invention pour son utilisation comme composition dermatologique, cosmétique, ou encore comme dispositif médical, avantageusement comme implant biorésorbable.

La présente invention a également pour objet l'utilisation cosmétique ou une méthode de traitement cosmétique ou esthétique du corps ou du visage humain comprenant l'injection d'une composition ou d'une solution aqueuse selon l'invention.

La présente invention a également pour objet l'utilisation d'une telle solution aqueuse en tant que vecteur de principes actifs, par exemple comme véhicule de vaccins, d'anticancéreux ou d'hormones.

Dans un mode de réalisation particulier, la composition ou la solution aqueuse selon la présente invention est destinée à être utilisée dans la réparation ou la reconstruction des tissus.

En particulier, la composition ou la solution aqueuse selon la présente invention peut être utilisée pour la constitution ou la substitution de tissus biologiques, par exemple comme implant, ou le comblement des tissus biologiques, par exemple l'injection dans les cartilages osseux ou dans les articulations ou pour le comblement des cavités du corps ou du visage, telles que les rides ou les ridules, pour la création ou l'augmentation de volumes du visage ou du corps humain, ou encore pour la cicatrisation de la peau.

Selon d'autres modes de réalisation particuliers, la composition ou la solution aqueuse selon la présente invention peut être utilisée :

- en chirurgie, notamment dans la réparation d'organes, ou en médecine ou chirurgie esthétique,
- en urologie, notamment pour le traitement de l'incontinence urinaire,
- en infectiologie, notamment comme fluide vecteur pour les vaccins,

- en ophtalmologie, notamment pour la cicatrisation cornéenne,
- en odontologie, notamment pour la pose d'un implant dentaire ou pour la réparation osseuse,
- en orthopédie, notamment dans le périoste pour la création de volume
ou encore en angiologie.

La composition ou la solution aqueuse selon la présente invention peut également être utilisée en rhumatologie.
Avantageusement, la composition ou la solution aqueuse selon la présente invention peut également être utilisée en tant que vecteur de principe actif notamment thérapeutique, tel qu'un vaccin ou une hormone du type insuline ou œstrogène, et d'une manière plus générale pour tous les principes actifs dont la délivrance ou libération contrôlée et/ou prolongée présente un avantage.
La présente invention concerne également l'utilisation cosmétique d'une solution aqueuse ou d'une composition selon l'invention pour traiter ou lutter contre le vieillissement de la peau.
Enfin, la présente invention concerne également une méthode pour augmenter la solubilité dans l'eau d'un chitosane ayant un degré d'acétylation inférieur à 20% et une masse moléculaire moyenne en masse comprise entre 100 000 et 1 500 000 g/mol tel que décrit précédemment, comprenant au moins les étapes suivantes :

- la dissolution du chitosane dans l'eau par ajout d'acide tel qu'un acide faible pour obtenir une solution aqueuse homogène de chitosane contenant, dans un milieu physiologiquement acceptable, un chitosane ayant un degré d'acétylation inférieur à 20% et une masse moléculaire moyenne en masse comprise entre 100 000 et 1 500 000 g/mol, ladite solution contenant entre 0,1 et 4,5 %, en poids de chitosane, ladite solution présentant un pH inférieur à 6,2,
ledit acide étant avantageusement choisi dans le groupe constitué par l'acide acétique, l'acide glycolique, l'acide lactique, l'acide glutamique, et leurs mélanges, et
- le réajustement du pH par dialyse, de préférence à température ambiante, pour obtenir une solution aqueuse présentant un pH supérieur ou égal à 6,2, avantageusement compris entre 6,2 et 7,2, de préférence entre 6,25 et 7,1.

Les étapes de la présente méthode étant décrites en détails précédemment.
Les exemples suivants sont destinés à illustrer l'invention sans aucunement en limiter la portée.

**Exemple 1 : Préparation d'une solution aqueuse de chitosane**

Solution acide de chitosane :

Une solution de chitosane aqueuse a été préparée par dissolution de chitosane solide dans de l'eau acidifiée par l'acide acétique.
Le chitosane utilisé est un chitosane de masse moléculaire Mw comprise entre 400 000 et 1 500 000 $g.mol^{-1}$ et degré d'acétylation = 5%.
Sa viscosité, mesurée à une concentration de 1% en solution aqueuse, avec 1% en acide acétique à 25°C, est de 9,578 Pa.s
1 ,5 g de chitosane a été dissous dans 500µL d'acide acétique glacial et 49,5mL de tampon PBS ("phosphate buffered saline" - pH=6,5) comprenant du NaCl, un sel « acide » $NaH_2PO_4$ et un sel «basique» le $Na_2HPO_4$.
Le pH de la solution est de 5,45.

Matériel de dialyse :

La dialyse a été réalisée à l'aide de membranes SpectraPor 4 en cellulose régénérée avec un seuil de ségrégation de 12-14000 Da.
Les pH sont mesurés à l'aide d'un pH-mètre portable FiveGo FG2 (Mettler Toledo).
Un tampon phosphate de type PBS ("phosphate buffered saline" - pH=6,5) a été utilisé.
La dialyse est effectuée contre 3L de tampon renouvelé régulièrement. La dialyse est arrêtée aux premiers signes de gélification.
Le pH de la solution de chitosane est mesuré régulièrement et son évolution est représentée sur la figure 2.
Le pH de la solution de chitosane a ainsi pu être augmenté jusqu'à une valeur de 6,46 sans aucun signe de gélification de la solution.

**Exemple 2 : Comparatif**

La demanderesse a tenté de reproduire la solution homogène de chitosane décrite dans l'exemple 1 de la demande de brevet WO03/042250.

Pour cela, un chitosane présentant un degré de désacétylation de 84% a été utilisé.

2,34 g de chitosane (désacétylé à 84%) a été dissout sous agitation dans 100 ml d'une solution de HCl (0. 1 M). Après 30 minutes d'agitation, une solution homogène de chitosane est obtenue.

La solution de chitosane a été refroidie au réfrigérateur à 4 °C et maintenue dans un bain de glace.

Son pH a été ajusté à 6,8 par addition, toujours à 4°C, d'un sel disodique d'αβ-glycéro-phosphate. 1,0g de sel disodique d'αβ-glycéro-phosphate a suffi pour atteindre le pH souhaité de 6,8.

Toutefois, les sels d'αβ-glycéro-phosphate se dispersaient très mal dans les solutions de chitosane malgré une agitation manuelle et mécanique. Des inclusions de gel sont apparues très rapidement autour des cristaux de glycéro-phosphate les moins solubles.

Ainsi, des solutions aqueuses homogènes de chitosane présentant un pH de 6,8 n'ont pas pu être obtenues en reproduisant l'exemple 1 du document WO03/042250.

Afin de résoudre ce problème, la demanderesse a reproduit cet essai en remplaçant le sel disodique d'αβ-glycéro-phosphate par un sel disodique de β-glycéro-phosphate, dont les propriétés d'amélioration de la solubilité du chitosane sont bien connues.

Pour ce nouvel essai, le même chitosane présentant un degré de désacétylation de 84% a été utilisé.

2,34 g de chitosane (désacétylé à 84%) a été dissout sous agitation dans 100 ml d'une solution de HCl (0. 1 M).

La solution de chitosane a été refroidie au réfrigérateur à 4 °C et maintenue dans un bain de glace.

Son pH a été ajusté à 6,8 par ajout progressif, toujours à 4°C, de 2,84g d'un sel disodique de β-glycéro-phosphate dispersés par agitation manuelle.

Une solution homogène de chitosane à 3,4°C, présentant un pH de 6,8, a ainsi été obtenue. La transmittance de la solution avant et après ajout de β-glycéro-phosphate a également été mesurée par diffusion de la lumière, à une longueur d'onde égale à 500 nm, à travers un échantillon de 1 cm d'épaisseur.

La solution a ensuite été chauffée au bain marie jusqu'à atteindre une température de 25°C (température ambiante), et a été maintenue à cette température pendant 17h. La composition obtenue est gélifiée et présente un aspect blanchâtre prononcé. La transmittance ne peut plus être mesurée compte tenu du caractère compact du gel obtenu.

Les résultats sont présentés dans le tableau ci-dessous :

| | Sans ajout de β-GP | Avec ajout de β-GP |
|---|---|---|
| **4°C (T=0)** | T°=3.4°C<br>pH=6.02<br>T%=100%<br>Liquide | T°=3.1°C<br>pH=6.81<br>T%=77%<br>Liquide |
| **25°C (17H)** | T°=24.5°C<br>pH=5.60<br>T%=100%<br>Liquide | T°=24.2<br>pH=6.81<br>T%= impossible gel trop compact.<br>Gel compact |

Ainsi, la solution obtenue à partir de l'exemple 1 du document WO03/042250, modifié par utilisation d'un sel disodique de β-glycéro-phosphate à la place d'un sel disodique d'αβ-glycéro-phosphate, est bien liquide et plutôt homogène (transmittance 77%) à basse température (4°C).

Toutefois, l'augmentation de température pour atteindre 25°C (température ambiante) entraine une gélification de la composition. Dans le document WO03/042250, c'est le greffage chimique du chitosane qui permet d'obtenir une solution injectable à 25°C. Sans ce greffage, les composition décrites dans le document WO03/042250 se présentent sous forme d'un gel dès 25°C et ne peuvent être injectables qu'à 4°C.

A l'inverse, les solutions aqueuses homogènes selon la présente invention se présentent sous forme de solution liquide injectable même à température ambiante (20-25°C), et ne gélifient que lors de l'augmentation du pH in situ.

## Revendications

1. Solution aqueuse homogène de chitosane injectable contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, ladite solution présentant un pH supérieur ou égal à 6,2, avantageusement compris entre 6,2 et 7,2, ladite solution ne contenant pas de chitosane ayant un degré d'acétylation supérieur à 20%, ladite solution étant liquide et homogène à la température ambiante.

2. Solution aqueuse selon la revendication précédente, **caractérisée en ce que** le chitosane a un degré d'acétylation inférieur à 19%, de préférence inférieur à 17%, plus avantageusement inférieur ou égal à 15%, et plus préférentiellement inférieur à 10%.

3. Solution aqueuse selon l'une quelconque des revendications précédentes, **caractérisée en ce qu'**elle contient entre 1 et 3,5 % en poids de chitosane, en particulier entre 2 et 3,5 %, en poids de chitosane, par rapport au poids total de la solution aqueuse.

4. Solution aqueuse selon l'une quelconque des revendications précédentes, **caractérisée en ce que** le chitosane a une masse moléculaire moyenne en masse comprise entre 200 000 et 1 000 000 g/mol, plus avantageusement entre 250 000 et 800 000 g/mol, et encore plus avantageusement entre 300 000 et 600 000 g/mol.

5. Solution aqueuse selon l'une quelconque des revendications précédentes, **caractérisée en ce qu'**elle présente un pH entre 6,25 et 7,1, de préférence entre 6,3 et 7,0.

6. Solution aqueuse selon l'une quelconque des revendications précédentes, **caractérisée en ce qu'**elle est susceptible d'être préparée par un procédé comprenant au moins les étapes suivantes :

   - la dissolution du chitosane dans l'eau par ajout d'acide tel qu'un acide faible pour obtenir une solution aqueuse homogène de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, , ladite solution présentant un pH inférieur à 6,2,
   ledit acide étant avantageusement choisi dans le groupe constitué par l'acide acétique, l'acide glycolique, l'acide lactique, l'acide glutamique, et leurs mélanges, et
   - le réajustement du pH par dialyse, de préférence à température ambiante, pour obtenir une solution aqueuse présentant un pH supérieur ou égal à 6,2, avantageusement compris entre 6,2 et 7,2, de préférence entre 6,25 et 7,1.

7. Solution aqueuse selon la revendication 3, **caractérisée en ce que** lors de l'étape de dissolution, l'acide est ajouté en une quantité strictement nécessaire à la dissolution du chitosane.

8. Solution aqueuse selon l'une des revendications 3 ou 4, **caractérisée en ce qu'**elle est dialysée contre une solution tampon présentant un pH supérieur à 6,2, avantageusement compris entre 6,2 et 7,2, de préférence entre 6,25 et 7,1.

9. Solution aqueuse selon l'une des revendications 3 à 5, **caractérisée en ce que** la solution tampon est une solution saline de tampon phosphate composée d'un sel « acide » $NaH_2PO_4$, d'un sel «basique » le $Na_2HPO_4$ et de NaCl.

10. Solution aqueuse selon l'une des revendications 3 à 6, **caractérisée en ce que** la dialyse est dynamique, mettant notamment en œuvre les étapes suivantes:

    i. la préparation, dans un premier réservoir A, d'une première solution comprenant un acide faible, appelée « solution acide faible »,
    ii. la préparation, dans un second réservoir B, d'une seconde solution comprenant la base conjuguée ou une base plus forte que la base conjuguée de l'acide faible utilisé dans la « solution acide faible »,
    iii. la circulation de la « solution acide faible » au travers d'une ou plusieurs poches de dialyse constituées d'une membrane de dialyse renfermant une solution aqueuse de chitosane contenant, dans un milieu physiologiquement acceptable, entre 0,1 et 4,5 %, en poids d'un chitosane ayant un degré d'acétylation inférieur à 20% et une masse moléculaire moyenne en masse comprise entre 100 000 et 1 500 000 g/mol, , ladite solution présentant un pH inférieur à 6,2,
    iv. l'introduction de ladite « solution acide faible » récupérée à l'issue de la dialyse de l'étape iii) dans le second

réservoir B, la composition dudit second réservoir B étant ainsi modifiée en un mélange « base + ε acide faible », ε représentant une quantité très minoritaire,

v. l'introduction du mélange « base + ε acide faible » du réservoir B dans le réservoir A pour obtenir un mélange « acide faible + ε base» dans ledit réservoir A,

vi. la répétition des étapes iii, iv, et v jusqu'à ce que l'équilibre acido-basique soit atteint dans les réservoirs A et B, formant ainsi, dans chaque réservoir, une solution tampon de pH déterminé.

**11.** Solution aqueuse selon la revendication 7, **caractérisée en ce que** l'acide faible mis en œuvre dans le réservoir A est un mélange de dihydrogénophosphate ($H_2PO_4$) et de NaCl et la base mise en œuvre dans le réservoir B est l'hydrogénophophate ($HPO_4^{2-}$).

**12.** Solution aqueuse selon l'une des revendications 3 à 8, **caractérisée en ce que** la membrane de dialyse est constituée d'un polymère de cellulose régénérée (cellulose naturelle transformée, par une suite d'opérations chimiques et physiques) ou d'un ester de cellulose (par exemple l'acétate de cellulose) avec un seuil d'exclusion compris entre 5kDa et 30KDa.

**13.** Composition comprenant une solution aqueuse selon l'une quelconque des revendications précédentes, et éventuellement un composé ou un excipent acceptable.

**14.** Composition selon la revendication 13, comprenant au moins un composé actif tel qu'un composé analgésique, anesthésique local, tel que la lidocaïne, mépivacaïne, bupivacaïne ou ropivacaïne, un composé angiogénique, un vaccin, une hormone, ou encore un composé actif du type facteur de croissance ou oligosaccharide bioactif, par exemple un oligosaccharide d'acide hyaluronique ou oligosaccharide de chitosane de degré de polymérisation inférieur à 20, ou encore un acide nucléique, une protéine ou un anticancéreux.

**15.** Composition selon la revendication 13 ou 14, **caractérisée en ce qu'**elle est formulée pour être administrée ou est utilisée par injection par voie intradermique ou sous-cutanée, intramusculaire, intra articulaire, intra oculaire, et de façon plus générale dans les tissus mous ou durs du corps par exemple les organes (appareil digestif ou uro-génital) ou les tissus adipeux, les muqueuses, les gencives, le cartilage, les os.

**16.** Composition selon l'une quelconque des revendications 13 à 15, pour son utilisation dans la réparation ou la reconstruction des tissus, en particulier dans la constitution ou la substitution de tissus biologiques, par exemple comme implant, ou le comblement des tissus biologiques, par exemple le comblement de dépression cutanée, l'injection dans les cartilages osseux ou dans les articulations.

**17.** Composition selon l'une quelconque des revendications 13 à 15, pour son utilisation comme composition dermatologique, cosmétique, ou encore comme dispositif médical, avantageusement comme implant biorésorbable.

**18.** Composition selon la revendication 17, pour son utilisation en chirurgie, en médecine ou chirurgie esthétique, en urologie, rhumatologie, ophtalmologie, odontologie, orthopédie, ou encore en angiologie.

**19.** Composition selon la revendication 17, pour son utilisation en tant que vecteur de principes actifs, par exemple comme véhicule de vaccins, d'anticancéreux ou d'hormones.

**Patentansprüche**

**1.** Injizierbare wässrige homogene Lösung von Chitosan enthaltend in einem physiologisch akzeptablen Milieu zwischen 0,1 und 4,5 Gewichts-% Chitosan, welches einen Acetylierungsgrad unterhalb von 20 % und eine molekulare Masse im Massenmittel umfasst zwischen 100.000 und 1.500.000 g/mol aufweist, wobei die Lösung einen pH oberhalb von oder gleich 6,2 aufweist, vorteilhafterweise umfasst zwischen 6,2 und 7,2, wobei die Lösung kein Chitosan enthält, welches einen Acetylierungsgrad oberhalb von 20 % aufweist, wobei die Lösung bei Umgebungstemperatur flüssig und homogen ist.

**2.** Wässrige Lösung gemäß dem vorstehenden Anspruch, **dadurch gekennzeichnet, dass** das Chitosan einen Acetylierungsgrad unterhalb von 19 % aufweist, vorzugsweise unterhalb von 17 %, stärker bevorzugt unterhalb oder gleich 15 % und am stärksten bevorzugt unterhalb von 10 %.

**3.** Wässrige Lösung gemäß einem der vorstehenden Ansprüche, **dadurch gekennzeichnet, dass** sie zwischen 1 und 3,5 Gewichts-% Chitosan enthält, insbesondere zwischen 2 und 3,5 Gewichts-% Chitosan in Bezug auf das Gesamtgewicht der wässrigen Lösung.

**4.** Wässrige Lösung gemäß einem der vorstehenden Ansprüche, **dadurch gekennzeichnet, dass** das Chitosan eine molekulare Masse im Massenmittel umfasst zwischen 200.000 und 1.000.000 g/mol aufweist, vorzugsweise zwischen 250.000 und 800.000 g/mol und noch stärker bevorzugt zwischen 300.000 und 600.000 g/mol.

**5.** Wässrige Lösung gemäß einem der vorstehenden Ansprüche, **dadurch gekennzeichnet, dass** sie einen pH zwischen 6,25 und 7,1 aufweist, vorzugsweise zwischen 6,3 und 7,0.

**6.** Wässrige Lösung gemäß einem der vorstehenden Ansprüche, **dadurch gekennzeichnet, dass** durch ein Verfahren herstellbar ist umfassend wenigstens die folgenden Schritte:

- Lösen von Chitosan in Wasser durch Hinzufügen von Säure wie einer schwachen Säure, um eine wässrige homogene Lösung von Chitosan zu erhalten, enthaltend in einem physiologisch akzeptablen Milieu zwischen 0,1 und 4,5 Gewichts-% Chitosan, welches einen Acetylierungsgrad unterhalb von 20 % und eine molekulare Masse im Massenmittel umfasst zwischen 100.000 und 1.500.000 g/mol aufweist, wobei die Lösung einen pH unterhalb von 6,2 aufweist,
wobei die Säure vorteilhafterweise ausgewählt wird aus der Gruppe bestehend aus Essigsäure, Glykolsäure, Milchsäure, Glutaminsäure und ihren Mischungen und
- Wiedereinstellen des pH durch Dialyse, vorzugsweise bei Umgebungstemperatur, um eine wässrige Lösung zu erhalten, welche einen pH oberhalb oder gleich von 6,2 aufweist, vorteilhafterweise umfasst zwischen 6,2 und 7,2, bevorzugt zwischen 6,25 und 7,1.

**7.** Wässrige Lösung gemäß Anspruch 3, **dadurch gekennzeichnet, dass** während des Lösungsschrittes die Säure in einer Menge hinzugefügt wird, welche absolut notwendig für die Lösung des Chitosans ist.

**8.** Wässrige Lösung gemäß einem der Ansprüche 3 oder 4, **dadurch gekennzeichnet, dass** sie gegen eine Pufferlösung dialysiert wird, welche einen pH oberhalb von 6,2 aufweist, vorteilhafterweise umfasst zwischen 6,2 und 7,2, bevorzugt zwischen 6,25 und 7,1.

**9.** Wässrige Lösung gemäß einem der Ansprüche 3-5, **dadurch gekennzeichnet, dass** die Pufferlösung eine Phosphatpuffer-Saline-Lösung, zusammengesetzt aus einem "*sauren*" Salz $NaH_2PO_4$, einem "*basischen*" Salz $Na_2HPO_4$ und NaCl ist.

**10.** Wässrige Lösung gemäß einem der Ansprüche 3-6, **dadurch gekennzeichnet, dass** die Dialyse dynamisch ist, wobei besonders die folgenden Schritte durchgeführt werden:

i. in einem ersten Behälter A die Herstellung einer ersten Lösung umfassend eine schwache Säure, welche "*Lösung der schwachen Säure*" genannt wird,
ii. in einem zweiten Behälter B die Herstellung einer zweiten Lösung umfassend eine konjugierte Base oder eine stärkere Base als die konjugierte Base der schwachen Säure, welche in der "*Lösung der schwachen Säure*" verwendet wurde,
iii. die Zirkulation der "*Lösung der schwachen Säure*" gegen eine oder mehreren Dialysebeutel, bestehend aus einer Dialysemembran, enthaltend eine wässrige Lösung von Chitosan enthaltend in einem physiologisch akzeptablen Milieu zwischen 0,1 und 4,5 Gewichts-% Chitosan, welches einen Acetylierungsgrad unterhalb von 20 % und eine molekulare Masse im Massenmittel umfasst zwischen 100.000 und 1.500.000 g/mol aufweist, wobei die Lösung einen pH unterhalb von 6,2 aufweist,
iv. das Einführen der "*Lösung der schwachen Säure*", gewonnen am Ende der Dialyse von Schritt iii in den zweiten Behälter B, wobei die Zusammensetzung des zweiten Behälters B so zu einer Mischung "*Base + $\varepsilon$ schwache Säure*" modifiziert wird, wobei $\varepsilon$ eine sehr kleine Menge darstellt,
v. das Einführen der Mischung *"Base + $\varepsilon$ schwache Säure"* des Behälters B in den Behälter A, um eine Mischung *"schwache Säure + $\varepsilon$ Base"* in dem Behälter A zu erhalten,
vi. die Wiederholung der Schritte iii, iv und v, bis ein Säure-Base-Gleichgewicht in den Behältern A und B erhalten wird, wobei so in jedem Reservoir eine Pufferlösung mit vorbestimmten pH gebildet wird.

**11.** Wässrige Lösung gemäß Anspruch 7, **dadurch gekennzeichnet, dass** die im Behälter A verwendete schwache

Säure eine Mischung von Dihydrogenphosphat ($H_2PO_4^-$) und NaCl ist und die im Behälter B verwendete Base Hydrogenphosphat ($HPO_4^{2-}$) ist.

**12.** Wässrige Lösung gemäß einem der Ansprüche 3-8, **dadurch gekennzeichnet, dass** die Dialysemembran aus einem regenerierten Cellulosepolymer (natürliche Zellulose, welche durch eine Folge von chemischen und physikalischen Schritten transformiert wurde) oder einem Celluloseester (z.B. Celluloseacetat) mit einer Ausschlussgrenze umfasst zwischen 5 kDa und 30 kDa gebildet wird.

**13.** Zusammensetzung umfassend eine wässrige Lösung gemäß einem der vorstehenden Ansprüche und gegebenenfalls eine akzeptable Verbindung oder ein akzeptables Excipiens.

**14.** Zusammensetzung gemäß Anspruch 13, umfassend wenigstens eine aktive Verbindung, wie eine analgetische Verbindung, ein Lokalanästhetikum, wie Lidocain, Mepivacain, Bupivacain oder Ropivacain, eine angiogenetische Verbindung, ein Vakzin, ein Hormon oder noch einen Wirkstoff vom Typ Wachstumsfaktor oder bioaktives Oligosaccharid, zum Beispiel Hyaluronsäure-Oligosaccharid oder Chitosan-Oligosaccharid mit einem Polymerisationsgrad unterhalb von 20, oder noch eine Nukleinsäure, ein Protein oder ein Anti-Krebsmittel.

**15.** Zusammensetzung gemäß Anspruch 13 oder 14, **dadurch gekennzeichnet, dass** sie formuliert ist zur Verabreichung oder verwendet wird zur Injektion über den intradermalen oder subkutanen, intramuskulären, intraartikulären, intraokularen Weg und auf allgemeinere Art in weiche oder harte Gewebe des Körpers, zum Beispiel Organe (Verdauungsapparat oder Urogenitaltrakt) oder Fettgewebe, Muskeln, Zahnfleisch, Knorpel, Knochen.

**16.** Zusammensetzung gemäß einem der Ansprüche 13-15, zur Verwendung zur Reparatur oder Rekonstruktion von Gewebe, insbesondere bei der Bildung oder beim Ersatz von biologischen Geweben, zum Beispiel als Implantat oder zum Füllen von biologischem Gewebe, zum Beispiel zum Füllen einer Hautvertiefung, Injektion in verknöcherte Knorpel oder in Gelenke.

**17.** Zusammensetzung gemäß einem der Ansprüche 13-15, zur Verwendung als dermatologische Zusammensetzung, kosmetische Zusammensetzung oder ebenso als medizinische Vorrichtung, vorteilhafterweise als bioresorbierbares Implantat.

**18.** Zusammensetzung gemäß Anspruch 17, zur Verwendung in der Chirurgie, in der kosmetischen Medizin oder Chirurgie, in der Urologie, Rheumatologie, Ophthalmologie, Odontologie, Orthopädie und außerdem in der Angiologie.

**19.** Zusammensetzung gemäß Anspruch 17, zur Verwendung als Vektor für Wirkstoffe, zum Beispiel als Vehikel für Vakzine, Anti-Krebsmittel oder Hormone.

## Claims

**1.** Injectable homogeneous aqueous solution of chitosan containing, in a physiologically acceptable medium, between 0.1 and 4.5% by weight of a chitosan having a degree of acetylation less than 20% and a weight average molecular mass of between 100,000 and 1,500,000 g/mol, said solution having a pH greater than or equal to 6.2, and advantageously between 6.2 and 7.2, said solution not containing any chitosan having a degree of acetylation greater than 20%, said solution being liquid and homogeneous at ambient temperature.

**2.** Aqueous solution according to the preceding claim, **characterised in that** the chitosan has a degree of acetylation less than 19%, preferably less than 17%, more advantageously less than or equal to 15%, and most preferably less than 10%.

**3.** Aqueous solution according to any one of the preceding claims, **characterised in that** it contains between 1 and 3.5 % by weight of chitosan, in particular between 2 and 3.5 % by weight of chitosan, with respect to the total weight of the aqueous solution.

**4.** Aqueous solution according to any one of the preceding claims, **characterised in that** the chitosan has a weight average molecular mass between 200,000 and 1,000,000 g/mol, more advantageously between 250,000 and 800,000 g/mol, and still more advantageously between 300,000 and 600,000 g/mol.

**5.** Aqueous solution according to any one of the preceding claims, **characterised in that** it has a pH between 6.25 and 7.1, preferably between 6.3 and 7.0.

**6.** Aqueous solution according to any one of the preceding claims, **characterised in that** it is able to be prepared by a method comprising the following steps:

- dissolving the chitosan in water by adding acid, such as a weak acid, in order to obtain a homogeneous aqueous solution of chitosan containing, in a physiologically acceptable medium, between 0.1 and 4.5% by weight of a chitosan having a degree of acetylation less than 20% and a weight average molecular mass of between 100,000 and 1,500,000 g/mol, said solution having a pH less than 6.2,
said acid being advantageously chosen from the group consisting of acetic acid, glycolic acid, lactic acid, glutamic acid, and the mixtures of same, and
- readjusting the pH by dialysis, preferably at ambient temperature, in order to obtain an aqueous solution having a pH greater than or equal to 6.2, advantageously between 6.2 and 7.2, and preferably between 6.25 and 7.1.

**7.** Aqueous solution according to claim 3, **characterised in that** during the dissolving step, the acid is added in a strictly necessary quantity for dissolving the chitosan.

**8.** Aqueous solution according to one of claims 3 or 4, **characterised in that** it is dialysed against a buffer solution having a pH greater than 6.2, advantageously between 6.2 and 7.2, preferably between 6.25 and 7.1.

**9.** Aqueous solution according to one of claims 3 to 5, **characterised in that** the buffer solution is a phosphate-buffered saline solution comprising an "acid" salt $NaH_2PO_4$, a "basic" salt $Na_2HPO_4$ and NaCl.

**10.** Aqueous solution according to one of claims 3 to 6, **characterised in that** the dialysis is dynamic, implementing in particular the following steps:

i. preparing, in a first reservoir A, a first solution comprising a weak acid, referred to as the "weak acid solution",
ii. preparing, in a second reservoir B, a second solution comprising the conjugate base or a base stronger than the conjugate base of the weak acid used in the "weak acid solution",
iii. circulating the "weak acid solution" through one or more dialysis pockets consisting of a dialysis membrane enclosing an aqueous solution of chitosan containing, in a physiologically acceptable medium, between 0.1 and 4.5% by weight of a chitosan having a degree of acetylation less than 20% and a weight average molecular mass of between 100,000 and 1,500,000 g/mol, said solution having a pH less than 6.2,
iv. introducing said "weak acid solution" recovered at the end of the dialysis of step iii) into the second reservoir B, the composition of said second reservoir B being thus modified to a "base + ε weak acid" mixture, ε representing a very small minority quantity,
v. introducing the "base + ε weak acid" mixture of reservoir B into reservoir A in order to obtain a "weak acid + ε base" mixture in said reservoir A,
vi. repeating steps iii, iv, and v until the acid-base equilibrium is attained in reservoirs A and B, thus forming, in each reservoir, a buffer solution having determined pH.

**11.** Aqueous solution according to claim 7, **characterised in that** the weak acid used in reservoir A is a mixture of dihydrogen phosphate ($H_2PO_4^-$) and NaCl and the base used in reservoir B is hydrogen phosphate ($HPO_4^{2-}$).

**12.** Aqueous solution according to one of claims 3 to 8, characterising that the dialysis membrane is made of a regenerated cellulose polymer (natural cellulose transformed by a series of chemical and physical operations) or a cellulose ester (for example cellulose acetate) with an exclusion threshold between 5 kDa and 30 kDa.

**13.** Composition comprising an aqueous solution according to any one of the preceding claims, and optionally a compound or an acceptable excipient.

**14.** Composition according to claim 13, comprising at least one active compound such as an analgesic compound, local anaesthetic, such as lidocaine, mepivacaine, bupivacaine or ropivacaine, an angiogenic compound, a vaccine, a hormone, or even an active compound such as a growth factor or bioactive oligosaccharide, for example an hyaluronic acid oligosaccharide or chitosan oligosaccharide having a degree of polymerisation less than 20, or even a nucleic acid, a protein or an anti-cancer agent.

**15.** Composition according to claim 13 or 14, **characterised in that** it is formulated to be administered or is used by intradermic or subcutaneous, intramuscular, intra-articular or intraocular injection, and more generally into the hard or soft tissue of the body, for example the organs (digestive system or urogenital system) or the adipose tissues, mucous membranes, gums, cartilage or bones.

**16.** Composition according to any one of claims 13 to 15, for use in the repair or reconstruction of tissues, in particular in the creation or substitution of biological tissues, for example as implants, or for the filling of biological tissues, for example filling of cutaneous depressions, injection into bone cartilage or into the joints.

**17.** Composition according to any one of claims 13 to 15, for use as dermatological or cosmetic composition, or for use as a medical device, advantageously as a bioresorbable implant.

**18.** Composition according to claim 17, for use in surgery, medicine or aesthetic surgery, in urology, rheumatology, ophthalmology, odontology, orthopaedics, or angiology.

**19.** Composition according to claim 17, for use as a vector for active substances, for example as a vehicle for vaccines, anti-cancer agents or hormones.

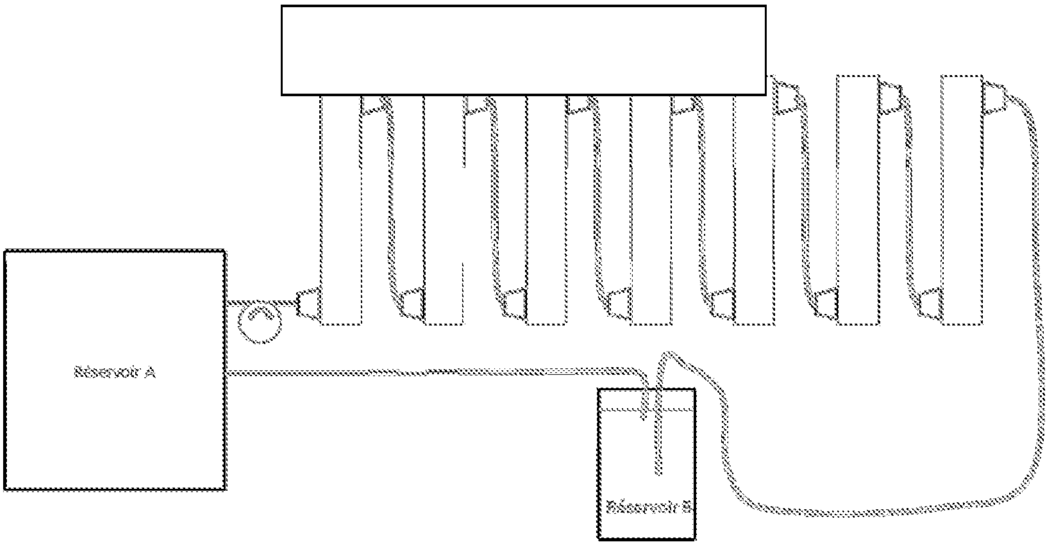


Figure 1

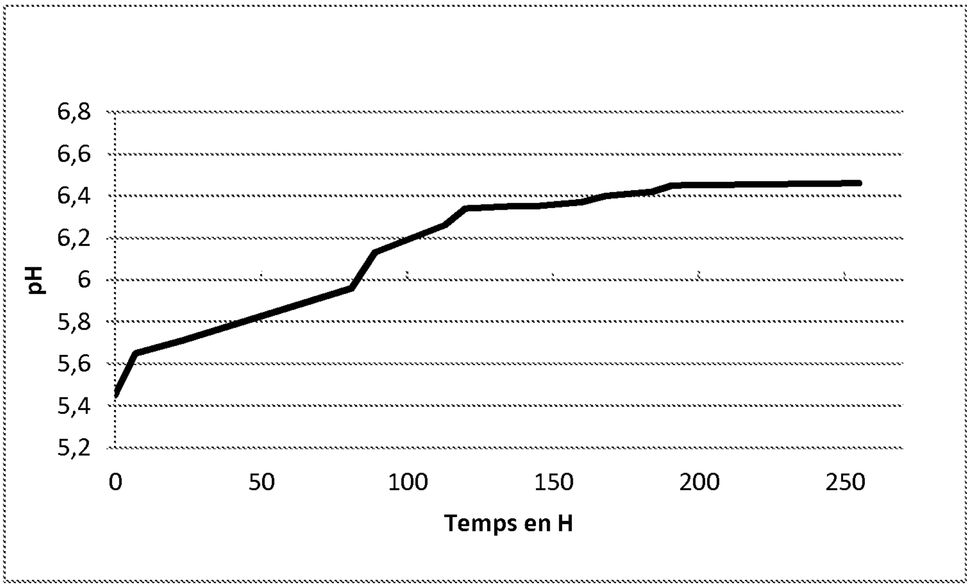


Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013079646 A
- WO 2009150651 A
- WO 03042250 A
- JP H0269502 B
- WO 2008072230 A


### Littérature non-brevet citée dans la description


- **A. MONTEMBAULT ; K. TAHIRI ; C. KORWIN-ZMIJOWSKA ; X, CHEVALIER ; M. CORVOL ; A.DOMARD.** *Biochimie,* 2006, vol. 88, 551-64
- **A. MONTEMBAULT ; C. VITON ; A. DOMARD.** Physico-chemical studies of the gelation of chitosan in a hydroalcoholic médium. *Biomaterials,* 2005, vol. 26 (8), 933-943
- **JAE HYUNG PARK et al.** Synthesis and characterization of sugar-bearing chitosan derivatives : aqueous solubility and biodegradability. *Biomacromolecules,* 2003, vol. 4, 1087-1091
- **VARUM K. M. et al.** Water solubility of partially N-acetylated chitosans as a function of pH : effect of chemical composition and depolymerization. *Carbohydrate polymers,* 1994, vol. 25, 65-70